# FASCICULE DE BREVET EUROPEEN

(11) **EP 0 927 051 B1**
(45) Date de publication et mention de la délivrance du brevet: **16.01.2002**
(21) Numéro de dépôt: 97919096.4
(22) Date de dépôt: 15.09.1997
(51) Int. Cl.: A61L 15/58

(54) **NOUVELLE MASSE ADHESIVE HYDROPHILE**
HYDROPHILES KLEBMITTEL
NOVEL HYDROPHILE ADHESIVE MASS

(30) Priorité: 16.09.1996 FR 9611249
(43) Date de publication de la demande: 07.07.1999
(73) Titulaire: LABORATOIRES D'HYGIENE ET DE DIETETIQUE, 75008 Paris (FR)
(72) Inventeur: AUGUSTE, Stéphane, F-21800 Quetigny (FR); APERT, Laurent, F-21000 Dijon (FR)
(74) Mandataire: Hubert, Philippe
(86) Numéro de dépôt international: FR9701621
(87) Numéro de publication internationale: WO9810801

(56) Documents cités:
- EP-A- 0 130 061
- EP-A- 0 297 769
- EP-A- 0 302 536
- EP-A- 0 730 874
- WO-A-91/06290
- DE-A- 2 032 268
- GB-A- 2 094 809

## Description

### Domaine de l'invention

La présente invention a pour objet de masses adhésives hydrophiles à base d'un copolymère séquencé du type poly(styrène-oléfine-styrène), et d'un hydrocolloïde auxquels est associé un polymère acrylate qui permet d'augmenter la capacité d'absorption de l'hydrocolloïde. L'invention concerne également l'utilisation de ces nouvelles masses adhésives à des fins médicales en particulier pour la réalisation de pansements protecteurs notamment de pansements anti-ampoules et de pansements pour le traitement des lésions dermo-épidermiques superficielles, plaies exsudatives et brûlures.

### Art antérieur

De nombreuses masses adhésives hydrophiles employées dans ces différentes applications médicales ont déjà été décrites. On peut ainsi citer par exemple les demandes de brevet suivantes, FR-A-2 495473, EP-A-130 061, EP-A-092 999, EP-A-302 536 ainsi que le brevet US-3 972 328.

Dans ces documents les masses adhésives hydrophiles décrites sont formées principalement d'un élastomère adhésif choisi parmi les polymères tels que les polyisobutènes ou des copolymères séquencés poly(styrène-oléfinestyrène) comme par exemple poly(styrène-isoprène-styrène) ou poly(styrèneéthylène-butylène-styrène), associé ou non à des résines poisseuses dites "tackifiantes", des plastifiants etc. et d'un ou plusieurs hydrocolloïdes.

L'élastomère adhésif de nature hydrophobe permet de conférer à la masse des propriétés d'élasticité, de déformation ou d'absorption des chocs (par exemple dans un pansement anti-ampoule) mais aussi de cohésion suffisante pour éviter le fluage de l'adhésif hors de la masse et permettre une maniabilité aisée. Mais il est incapable d'absorber les liquides ce qui conduit à des produits qui collent difficilement sur peau humide et ne collent pas ou se décollent rapidement dès l'absorption de sueur ou d'exsudats.

L'hydrocolloïde grâce à son caractère hydrophile confère à ces masses adhésives leurs propriétés d'absorption de l'eau, de la sueur, des liquides ou exsudats. Mais il a tendance alors à gonfler ce qui entraîne une perte de cohésion et conduit au démantèlement du pansement lors du retrait voire à sa perte.

L'association des propriétés de l'élastomère adhésif et de l'hydrocolloïde ne suffit pas pour répondre de façon satisfaisante aux nombreuses exigences souvent contradictoires requises, dans le cadre des applications médicales citées ci-dessus, pour de telles masses adhésives hydrophiles. De plus, sachant qu'il faut aussi tenir compte des possibilités de combinaison des composés entrant dans la formulation de la masse, la marge de manoeuvre pour la réalisation à des fins médicales d'une masse adhésive hydrophile présentant une absorption maximale et une adhésion durable dans le temps est très étroite. Enfin il faut encore ajouter des contraintes plus spécifiques pour ces produits qui doivent par exemple coller sur la peau mais pas sur la plaie, protéger la plaie mais éviter les problèmes d'irritation et de macération etc.

Les diverses formulations et les divers additifs spécifiques pour améliorer l'efficacité du couple élastomère adhésif-hydrocolloïde proposés dans les documents cités précédemment illustrent la nécessité et la difficulté d'optimiser les propriétés d'absorption sans nuire aux propriétés d'adhésion et en tenant compte de toutes les exigences précitées pour obtenir des produits plus performants et élargir les possibilités de formulation.

Mais aucune des publications énoncées ci-dessus ne divulgue ni ne suggère les nouvelles masses adhésives hydrophiles spécifiques de l'invention qui permettent d'apporter une nouvelle solution performante et inattendue, tant en termes d'absorption que d'adhésion, aux exigences et aux problèmes posés par la réalisation de masses adhésives hydrophiles à des fins médicales.

### Buts de l'invention

Dans le domaine des masses adhésives hydrophiles employées à des fins médicales il serait donc souhaitable de disposer d'une nouvelle solution technique permettant de réaliser un compromis entre les exigences précitées.

Selon un premier aspect de l'invention, on se propose de fournir une masse adhésive hydrophile dans laquelle on ajoute un polymère acrylate à un hydrocolloïde et un copolymère séquencé styrène-oléfine-styrène notamment un poly(styrène-isoprène-styrène).

Selon un second aspect de l'invention on propose l'utilisation de cette nouvelle masse adhésive hydrophile pour la réalisation de pansements protecteurs notamment de pansements pour le traitement de l'ampoule, le traitement des lésions dermo-épidermiques superficielles, plaies exsudatives et brûlures.

### Objet de l'invention

Les buts précités sont atteints grâce à une nouvelle solution technique qui consiste en la réalisation d'une masse adhésive hydrophile utilisable à des fins médicales, caractérisée en ce que ladite masse adhésive hydrophile comprend :
**(a)** 10 à 35 parties en poids d'un copolymère séquencé styrène-oléfine-styrène notamment un poly(styrène-isoprène-styrène),
**(b)** 20 à 50 parties en poids d'une résine tackifiante,
**(c)** 2 à 15 parties en poids d'un polymère acrylate ayant une température de transition vitreuse inférieure à -20 °C,
**(d)** 2 à 25 parties en poids d'un plastifiant, notamment d'une huile plastifiante,
**(e)** 20 à 50 parties en poids d'un hydrocolloïde,
**(f)** 0,1 à 2 parties en poids d'au moins un agent antioxydant.

Selon un mode de réalisation actuellement préféré, cette masse adhésive hydrophile comprend, pour un total de 100 parties en poids :
- 18 à 22, et de préférence 19,8 parties en poids d'un copolymère tribloc poly(styrène-isoprène-styrène),
- 25 à 35, et de préférence 30,2 parties en poids d'une résine tackifiante,
- 3 à 8, et de préférence 4,9 parties en poids d'un copolymère d'acrylate de n-butyle et d'acide acrylique qui présente une température de transition vitreuse de -39 °C,
- 10 à 20, et de préférence 14,3 parties en poids d'une huile plastifiante minérale,
- 25 à 35, et de préférence 30 parties en poids de carboxyméthylcellulose de sodium,
- 0,3 à 0,8, et de préférence 0,4 parties en poids d'un agent antioxydant phénolique, et,
- 0,3 à 0,8, et de préférence 0,4 parties en poids d'un agent antioxydant soufré, le dibutyldithiocarbamate de zinc.

On préconise l'utilisation de la masse adhésive hydrophile pour la réalisation de pansements protecteurs en particulier pour le traitement de l'ampoule, des lésions dermo-épidermiques superficielles de la peau, des plaies exsudatives et des brûlures.

Les copolymères séquencés du type styrène-oléfine-styrène qui sont susceptibles d'être utilisés dans le cadre de la présente invention sont ceux habituellement utilisés par l'homme de l'art dans la préparation de masse adhésive et l'on pourra se reporter à cet égard au document de l'état de la technique mentionné précédemment.

Les séquences oléfines de ces copolymères peuvent être constituées d'unités isoprène, butadiène ou éthylène-butylène.

Parmi ceux-ci, les copolymères triblocs poly(styrène-isoprène-styrène) sont préférés.

Par copolymère tribloc poly(styrène-isoprène-styrène) [en abrégé : poly(SIS)] on entend ici un matériau poly(SIS) ayant une teneur en styrène comprise entre 14 et 52 % en poids par rapport au poids dudit poly(SIS). Cette expression couvre aussi des poly(SIS) contenant un mélange de copolymères tribloc poly(SIS) et de copolymères dibloc du type poly(styrène-isoprène).

De tels produits bien connus de l'homme de l'art sont par exemple commercialisés par les sociétés SHELL et EXXON CHEMICAL respectivement sous les dénominations KRATON® D et VECTOR® .

Dans le cadre de la présente invention on préfère les copolymères tribloc ayant une teneur en styrène comprise entre 14 et 30 % en poids par rapport au poids dudit poly(SIS). On préférera en particulier les produits commercialisés respectivement sous les dénominations VECTOR® 4114 par la société EXXON CHEMICAL et KRATON® D-1111CS par la société SHELL CHEMICALS.

Parmi les résines tackifiantes qui conviennent selon l'invention, on peut mentionner les résines généralement employées dans le domaine des adhésifs par l'homme de l'art comme les résines polyterpènes ou terpènes modifiées, les résines de colophane hydrogénée, les résines de colophane polymérisée, les résines d'esters de colophane, les résines hydrocarbonées, les mélanges de résines aromatiques et aliphatiques etc. On préférera en particulier dans le cadre de la présente invention une résine de synthèse formée de copolymères en C₅/C₉ commercialisée par la société GOOD YEAR sous la dénomination WINGTACK® 86.

Par agents antioxydants on entend désigner ici les composés couramment employés par l'homme de l'art pour assurer la stabilité vis à vis de l'oxygène, la chaleur, l'ozone et les rayonnements ultra violets des composés utilisés dans la formulation de la masse adhésive hydrophile, en particulier les résines tackifiantes et les copolymères séquencés. On peut utiliser un ou plusieurs de ces agents antioxydants en association.

On peut citer ainsi comme agents antioxydants appropriés les antioxydants phénoliques comme par exemple les produits commercialisés par la société CIBA GEIGY sous les dénominations IRGANOX® 1010, IRGANOX® 565, IRGANOX® 1076 et des antioxydants soufrés comme par exemple le dibutyldithiocarbamate de zinc commercialisé par la société AKZO sous la dénomination PERKACIT® ZDBC.

Dans le cadre de la présente invention, on préférera l'association de l'IRGANOX® 1010 et du PERKACIT® ZDBC.

Par hydrocolloïde on entend désigner ici les composés couramment utilisés par l'homme de l'art, connus pour leur aptitude à absorber les liquides hydrophiles, en particulier l'eau et les transporter rapidement. Comme hydrocolloïdes appropriés, on peut citer par exemple l'alcool polyvinylique, la gélatine, la pectine, les alginates de sodium, les gommes végétales naturelles telles que la gomme de caroube, la gomme de Karaya, la gomme guar, la gomme arabique..., les dérivés de cellulose tels que les hydroxyéthylcelluloses, les hydroxypropylcelluloses, les carboxyméthylcelluloses et leurs sels de métal alcalin, tel le sodium ou le calcium. On pourra utiliser ces hydrocolloïdes seuls ou en association.

Dans le cadre de la présente invention on préférera les sels de métal alcalin de carboxyméthylcellulose; en particulier la carboxyméthylcellulose de sodium.

Tout type de plastifiant habituellement utilisé par l'homme de l'art pour la préparation de masse adhésive à base de copolymère séquencé styrène-oléfine-styrène peut être utilisé dans le cadre de la présente invention. On préféra cependant utiliser des huiles plastifiantes.

Par huiles plastifiantes on entend désigner ici les huiles minérales ou végétales couramment employées par l'homme de l'art pour plastifier les copolymères séquencés du type styrène-oléfine-styrène utilisés dans la composition de la masse adhésive.

Les huiles minérales généralement utilisées sont des mélanges de composés de nature paraffinique, naphténique ou aromatique dans des proportions variables.

On peut citer ainsi comme exemple d'huiles plastifiantes les produits commercialisés par la société SHELL sous la dénomination ONDINA® et RISELLA® pour les mélanges à base de composés naphténiques et paraffiniques ou sous la dénomination CATENEX® pour les mélanges à base de composés naphténiques, aromatiques et paraffiniques.

Dans le cadre de la présente invention on préférera en particulier l'huile plastifiante minérale commercialisée sous la dénomination CATENEX® N945.

Les polymères acrylates convenant pour la mise en oeuvre de l'invention sont des composés d'acrylates sensibles à la pression et ayant une température de transition vitreuse (Tg) inférieure à -20 °C.

De tels composés d'acrylates sont des copolymères formés
- soit d'au moins un monomère, choisi dans l'ensemble constitué par les esters alkylés de l'acide acrylique dans lesquels le groupe alkyle, linéaire ou ramifié, de l'ester comprend 1 à 18 atomes de carbone de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle, d'isooctyle, de n-décyle et n-dodécyle, copolymérisé avec l'acide acrylique;
- soit d'au moins deux monomères, choisis dans l'ensemble constitué par les esters alkylés de l'acide acrylique dans lesquels le groupe alkyle, linéaire ou ramifié, de l'ester comprend 1 à 18 atomes de carbone de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle, d'isooctyle, de n-décyle et n-dodécyle.

Les pourcentages ou proportions respectifs de ces différents monomères sont ajustés de façon à obtenir un copolymère ayant la température de transition vitreuse souhaitée c'est à dire inférieure à -20 °C.

Dans le cadre de la présente invention, on utilisera de préférence un copotymère contenant au moins un monomère, choisi parmi l'acrylate de n-butyle, l'acrylate de 2-éthylhexyle et l'acrylate d'isooctyle, copolymérisé avec l'acide acrylique.

On préférera tout particulièrement les copolymères contenant de 1 à 20 %, de préférence 1 à 10 %, en poids d'acide acrylique, exprimé par rapport au poids total de l'ensemble des monomères.

De tels composés d'acrylates peuvent aussi être des homopolymères dont le monomère constitutif est choisi dans l'ensemble constitué par les esters alkylés de l'acide acrylique dans lesquels le groupe alkyle de l'ester est soit un groupe alkyle linéaire qui comprend 2 à 12 atomes de carbone soit un groupe isobutyle, 2-éthylhexyle ou isooctyle.

Parmi ces homopolymères, on préférera dans le cadre de la présente invention, le polyacrylate de n-butyle.

Selon une caractéristique particulière de l'invention, on choisira les produits bien connus de l'homme de l'art pour être utilisables dans un procédé d'enduction sans solvant connu sous le nom "hot-melt".

On peut ainsi citer par exemple les produits commercialisés par la société BASF sous les dénominations suivantes:
- ACRONAL® A150F (homopolymère d'acrylate de n-butyle qui possède une température de transition vitreuse de -41 °C),
- ACRONAL® DS3435X (homopolymère d'acrylate de n-butyle qui possède une température de transition vitreuse de -46°C),
- ACRONAL® DS3429 (copolymère d'acrylate de n-butyle, d'acrylate de 2-éthylhexyle et d'acide acrylique qui possède une température de transition vitreuse de -31 °C), et
- ACRONAL® DS3458 (copolymère d'acrylate de n-butyle et d'acide acrylique qui possède une température de transition vitreuse de -39 °C.)

On peut citer également le produit commercialisé par la société MONSANTO sous la dénomination :
- MODAFLOW® (copolymère d'acrylate d'éthyle et d acrylate de 2-éthylhexyle).

La masse adhésive hydrophile selon l'invention est utile pour de nombreuses applications à des fins médicales. On peut ainsi citer la réalisation de bandages et de pansements pour la protection de la peau, pour la protection des cors, durillons, oeils de perdrix et pour le traitement des lésions dermo-épidermiques superficielles, piqûres d'insectes, plaies exsudatives, escarres et brûlures ainsi que le traitement et la prévention de l'ampoule.

De nombreuses autres applications telles la réalisation de joints adhésifs employés en ostomies, de dispositifs orthopédiques pour absorber les chocs dans les chaussures, de draps chirurgicaux, d'adhésifs pour électrodes dans le cadre du traitement du corps humain par un courant électrique et d'adhésifs pour fixer des prothèses ou des dispositifs sur la peau ou les membranes muqueuses, sont aussi possibles.

Dans le cadre de ces applications divers produits à caractère dermatologique ou thérapeutique peuvent être ajoutés dans la formulation de la masse adhésive hydrophile comme par exemple des antifongiques, des anti-microbiens ou antibactériens telle la sulfadiazine argentique, des régulateurs de pH, des accélérateurs de cicatrisation, des vitamines, des oligo-éléments, des anesthésiques locaux, du menthol, du salicylate de méthyle, des hormones, des anti-inflammatoires etc.

Dans le cadre de la réalisation d'un pansement pour le traitement de l'ampoule ou la protection des plaies, brûlures et escarres, on réalise une enduction de la masse adhésive hydrophile selon l'invention sur un support adéquat au grammage souhaité, selon les techniques connues de l'homme de l'art, suivant un procédé en phase solvant ou de préférence suivant un procédé hot-melt à une température généralement comprise entre 110 et 160 °C.

Le choix du support est réalisé en fonction des propriétés requises (étanchéité, élasticité etc.) suivant le type de pansement et l'application recherchée.

Il peut se présenter sous forme d'un film formé d'une ou plusieurs couches et d'une épaisseur variable de 5 à 150 µm ou d'un non-tissé ou d'une mousse ayant une épaisseur de 10 à 500 µm.

Ces supports à base de matériaux synthétiques ou naturels sont ceux généralement utilisés par l'homme de l'art dans le domaine des pansements et des applications médicales visées ci-dessus.

On peut citer ainsi des mousses en polyéthylène, en polyuréthanne, en PVC ; des non-tissés en polypropylène, polyamide, polyester, éthylcellulose etc...

On préférera cependant utiliser des films en tant que supports et notamment des films en polyuréthanne comme par exemple les produits commercialisés par la société Smith et Nephew sous la référence LASSO ou des films en polyuréthanne réalisés à partir du polyuréthanne commercialisé sous la dénomination ESTANE® par la société BF GOODRICH, des films en polyéthylène basse densité comme par exemple ceux commercialisés par la société SOPAL, des films à base de copolymère thermoplastique de polyéther-polyester comme par exemple les produits commercialisés sous la marque Hytrel® par la société DUPONT DE NEMOURS ou des films complexes réalisés à base de polyuréthanne et d'un non-tissé.

Les pansements réalisés à partir de la masse adhésive hydrophile selon l'invention peuvent se présenter sous n'importe quelle forme géométrique, carrée, rectangulaire, circulaire ou ovale. De même leur taille peut être quelconque et sera adaptée en fonction de la surface de la partie à traiter ou protéger.

De façon pratique, la surface de la masse adhésive qui n'est pas liée au support, pourra être recouverte d'une couche ou pellicule de protection pelable avant utilisation du pansement.

L'ensemble ainsi formé pourra être lui-même emballé dans une protection étanche réalisée par exemple au moyen de complexes polyéthylènealuminium ou dans des blisters.

Grâce à la spécificité de la composition des formulations pouvant former la masse adhésive hydrophile selon l'invention cette dernière présente de nombreux avantages que l'on va maintenant exposer.

Il a en effet été trouvé que, de façon surprenante, l'ajout, aux masses adhésives connues de l'art antérieur, d'un polymère acrylate, qui est un composé très visqueux de nature oléophilique peu ou pas compatible avec les liquides hydrophiles permet d'augmenter de façon significative la capacité d'absorption des masses adhésives à base d'hydrocolloïdes. Ce phénomène inattendu pourrait être le résultat d'une synergie entre l'hydrocolloïde et une certaine hydrophilie qui serait conférée au polymère par le caractère polaire des liaisons esters des acrylates. Or dans le cadre des applications médicales visées lors de l'utilisation des masses adhésives hydrophiles, l'augmentation du pouvoir absorbant est un critère déterminant.

En effet on ne peut obtenir ce résultat en augmentant de façon indéfinie la quantité d'hydrocolloïde car au-delà d'une certaine valeur l'hydrocolloïde altère les propriétés adhésives de la masse, joue sur les propriétés physiques notamment la cohésion, la capacité à la déformation, l'élasticité etc. On a aussi des problèmes de compatibilité entre les composés hydrophobes comme les SIS et hydrophiles comme l'hydrocolloïde et il devient impossible d'obtenir un produit homogène et d'aspect correct. L'ajout du polymère acrylate en quantité relativement faible par rapport à celle de l'ensemble élastomère-résine d'une part et hydrocolloïde d'autre part permet d'éviter ces problèmes. Il permet aussi de réaliser le compromis difficile à atteindre entre absorption maximale et propriétés adhésives et physiques acceptables, indispensable pour les applications à visée médicale souhaitées.

On évite ou minimise ainsi les phénomènes de perte ou de désintégration d'un pansement après gonflement et saturation par des fluides dans le cadre du traitement des plaies superficielles, exsudatives et des brûlures. On a moins de risques de macération, d'irritation et donc on peut renouveler ce dernier de façon indolore et surtout moins souvent.

Cette possibilité de soins facilités et espacés permet donc un traitement et une cicatrisation mieux contrôlés.

Enfin la masse adhésive hydrophile selon l'invention malgré l'augmentation de sa capacité d'absorption conserve son pouvoir adhésif sur la peau au cours du temps et même possède un pouvoir adhésif supérieur à celui d'une masse identique classique sans ajout du polymère acrylate.

Ceci permet par exemple de réaliser un pansement pour le traitement de l'ampoule qui s'adapte et colle efficacement sur la partie à protéger et qui permet grâce à son pouvoir absorbant de prévenir l'ampoule et d'obtenir un excellent produit hyppoallergénique.

D'autres avantages, caractéristiques et applications de l'invention seront mieux compris à la lecture de la description qui va suivre d'exemples de réalisation et d'essais comparatifs.

Bien entendu l'ensemble de ces éléments n'est nullement limitatif mais est donné à titre d'illustration.

Par commodité, dans ce qui suit les abréviations suivantes ont été utilisées :
SIS : copolymère tribloc poly(styrène-isoprène-styrène).

### Exemple 1

Dans un malaxeur à bras en Z, à une température de l'ordre de 130 °C on introduit successivement 14,2 kg de CATENEX® N945 (huile minérale commercialisée par la société SHELL), 19,8 kg de VECTOR® 4114 (copolymère SIS commercialisé par la société DEXCO), 0,4 kg de PERKACIT® ZDBC (dibutyl dithiocarbamate de zinc, antioxydant commercialisé par la société AKZO) et 0,4 kg d'IRGANOX® 1010 (antioxydant commercialisé par la société CIBA-GEIGY). On malaxe le mélange obtenu à 130 °C durant 35 minutes. On ajoute ensuite 4,9 kg d'ACRONAL® DS 3458 (copolymère de butylacrylate et d'acide acrylique commercialisé par la société BASF) et on malaxe le mélange obtenu toujours à 130 °C durant 10 minutes. On introduit alors 30,2 kg de WINGTACK® 86 (résine tackifiante commercialisée par la société GOOD YEAR) et on malaxe toujours à 130 °C durant 20 minutes supplémentaires. On introduit finalement 30 kg de BLANOSE® 7H4XF (carboxyméthylcellulose de sodium, commercialisé par la société AQUALON) et on malaxe encore durant 25 minutes. On enduit le mélange ainsi obtenu sur une pellicule de papier siliconé à raison de 600 g/m² à une température comprise entre 120 et 150 °C. On transfère l'enduction ainsi réalisée sur un support final, de 30 µm d'épaisseur, en polyuréthanne (réalisé à partir de polyuréthanne commercialisé sous la dénomination ESTANE® par la société BF GOODRICH). On découpe alors des formes aux dimensions appropriées que l'on conditionne dans des sachets thermosoudables ou des *blisters.*

### Exemple 2

On procède de façon analogue à l'exemple 1 mais dans ce cas on enduit sur la pellicule de papier siliconé à raison de 300 g/m².

### Exemple 3

On procède de façon analogue à l'exemple 1 mais on utilise dans ce cas une carboxyméthylcellulose de sodium de granulométrie différente. On remplace donc ici 30 kg de BLANOSE® 7H4XF par la même quantité de BLANOSE® 7H3XF (produit aussi commercialisé par la société AQUALON).

### Exemple 4

On procède de façon identique à l'exemple 1 mais dans ce cas on utilise 14,8 kg de CATENEX® N945, 21,1 kg de VECTOR® 4114, 0,42 kg de PERKACIT® ZDBC, 0,42 kg d'IRGANOX® 1010, 5 kg d'ACRONAL® DS 3458, 31,6 kg de WINGTACK® 86 et 26,6 kg de BLANOSE® 7H4XF. On enduit toujours à 600 g/m².

### Exemple 5

On procède de façon identique à l'exemple 1 mais on emploie dans ce cas un copolymère SIS différent. On utilise dans ce cas 14,8 kg de CATENEX® N945, 21,1 kg de KRATON® D-1111CS (copolymère SIS commercialisé par la société SHELL Chemicals), 0,42 kg de PERKACIT® ZDBC, 0,42 kg d'IRGANOX® 1010, 5,1 kg d'ACRONAL® DS 3458, 31,6 kg de WINGTACK® 86 et 26,6 kg de BLANOSE® 7H4XF. On enduit cette fois-ci sur une pellicule de papier siliconé à raison de 1000 g/m² et on transfère l'enduction obtenue sur un support final, de 30 µm d'épaisseur, en polyuréthanne commercialisé sous la référence LASSO 687 par la société Smith et Nephew.

### Exemple 6

On procède de façon analogue à l'exemple 5 mais dans ce cas on emploie un homopolymère d'ester acrylique l'ACRONAL® A150F à la place de l'ACRONAL® DS 3458. On utilise dans ce cas 14,2 kg de CATENEX® N945, 20,2 kg de KRATON® D-1111CS, 0,4 kg de PERKACIT® ZDBC, 0,4 kg d'IRGANOX® 1010, 4,9 kg d'ACRONAL® A150F (acrylate de n-butyle commercialisé par la société BASF), 34,4 kg de WINGTACK® 86 et 26 kg de BLANOSE® 7H4XF. On enduit cette fois-ci sur une pellicule de papier siliconé à raison de 600 g/m² et on transfère l'enduction obtenue sur un support final, de 30 µm d'épaisseur, en polyuréthanne (formé à partir d'ESTANE® ) identique à celui de l'exemple 1.

### Exemple 7

On procède de façon analogue à l'exemple 5 mais on remplace ici l'ACRONAL® DS 3458 par l'ACRONAL® DS 3429 qui est un copolymère de n-butylacrylate, 2-éthylhexylacrylate et d'acide acrylique commercialisé par la société BASF. On utilise dans ce cas 14,5 kg de CATENEX® N945, 20,6 kg de KRATON® D-1111CS, 0,41 kg de PERKACIT® ZDBC, 0,41 kg d'IRGANOX® 1010, 3 kg d'ACRONAL® DS 3429, 35,1 kg de WINGTACK® 86 et 26 kg de BLANOSE® 7H4XF. On enduit cette fois-ci sur une pellicule de papier siliconé à raison de 600 g/m² et on transfère l'enduction obtenue sur un support final, de 30 µm d'épaisseur, en polyéthylène basse densité commercialisé par la société SOPAL.

### Exemple 8

On procède de façon analogue à l'exemple 7 mais on emploie dans ce cas 8 % d'ACRONAL® DS 3429 au lieu de 3 % dans l'exemple 7 et on transfère sur un support final en polyéther-polyester. On utilise donc dans ce cas 13,7 kg de CATENEX® N945, 19,6 kg de KRATON® D-1111CS, 0,39 kg de PERKACIT® ZDBC, 0,39 kg d'IRGANOX® 1010, 8 kg d'ACRONAL® DS 3429, 33,3 kg de WINGTACK® 86 et 24,7 kg de BLANOSE® 7H4XF. On enduit toujours sur une pellicule de papier siliconé à raison de 600 g/m² mais on transfère ici l'enduction obtenue sur un support final, de 30 µm d'épaisseur, formé d'un copolymère thermoplastique polyéther-polyester commercialisé sous la dénomination Hytrel® par la société DUPONT DE NEMOURS.

### Exemple comparatif 1

Dans un malaxeur à bras en Z, à une température de l'ordre de 130 °C on introduit successivement 15 kg de CATENEX® N945, 20,8 kg de VECTOR® 4114, 0,4 kg de PERKACIT® ZDBC et 0,4 kg d'IRGANOX® 1010. On malaxe le mélange obtenu à 130 °C durant 35 minutes. On introduit alors 31,8 kg de WINGTACK® 86 et on malaxe toujours à 130 °C durant 20 minutes supplémentaires. On ajoute finalement 31,6 kg de BLANOSE® 7H4XF et on malaxe encore durant 25 minutes. On enduit le mélange ainsi obtenu sur une pellicule de papier siliconé à raison de 600 g/m² à une température comprise entre 120 et 150 °C. On transfère l'enduction ainsi réalisée sur un support final, de 30 µm d'épaisseur, en polyuréthanne (formée à partir d'ESTANE® ) identique à celui de l'exemple 1. On découpe alors des formes aux dimensions appropriées que l'on conditionne dans des sachets thermosoudables ou des blisters.

### Exemple comparatif 2

On procède de façon analogue à l'exemple comparatif 1 mais on enduit dans ce cas à raison de 300 g/m².

### Exemple comparatif 3

On procède de façon analogue à l'exemple comparatif 1 mais on emploie dans ce cas une carboxyméthylcellulose de sodium de granulométrie différente. On remplace le BLANOSE® 7H4XF par la même quantité de BLANOSE® 7H3XF. On enduit toujours à 600 g/ m² et on transfère sur le même support final.

### Exemple comparatif 4

On procède de façon analogue à l'exemple comparatif 1 mais on utilise dans ce cas 15,6 kg de CATENEX® N945, 22,2 kg de VECTOR® 4114, 0,44 kg de PERKACIT® ZDBC et 0,44 kg d'IRGANOX® 1010, 33,3 kg de WINGTACK ® 86 et 28 kg de BLANOSE® 7H4XF.

### Exemple comparatif 5

On procède de façon analogue à l'exemple comparatif 4 mais on remplace le VECTOR® 4114 par la même quantité de KRATON® D-1111CS. En revanche on enduit cette fois-ci sur une pellicule de papier siliconé à raison de 1000 g/m² et on transfère l'enduction ainsi obtenue sur un support final, de 30 µm d'épaisseur, en polyuréthanne commercialisé sous la référence LASSO 687 par la société Smith et Nephew.

### Exemple comparatif 6

On procède de façon analogue à l'exemple comparatif 5 mais on utilise dans ce cas 14,9 kg de CATENEX® N945, 21,3 kg de KRATON® D-1111CS, 0,42 kg de PERKACIT® ZDBC et 0,42 kg d'IRGANOX® 1010, 36,2 kg de WINGTACK® 86 et 26,8 kg de BLANOSE® 7H4XF. On enduit à raison de 600 g/m² sur une pellicule de papier siliconé et on transfère l'enduction ainsi obtenue sur un support final en polyuréthanne identique à celui de l'exemple 1.

### Exemple comparatif 7

On procède de façon analogue à l'exemple comparatif 5 mais dans ce cas on transfère l'enduction obtenue sur un support final, de 30 µm d'épaisseur, en polyéthylène basse densité commercialisé par la société SOPAL.

### Exemple comparatif 8

On procède de façon analogue à l'exemple comparatif 5 mais dans ce cas on transfère l'enduction obtenue sur un support final, de 30 µm d'épaisseur, formé d'un copolymère thermoplastique polyéther-polyester commercialisé sous la dénomination HYTREL® par la société DUPONT DE NEMOURS.

### Essais

Afin de mettre en évidence la capacité d'absorption des masses adhésives hydrophiles selon l'invention on a réalisé des mesures d'absorption avec différents échantillons obtenus à partir des exemples 1 à 8.

Dans un but comparatif on a déterminé de la même façon l'absorption de masses adhésives hydrophiles identiques mais sans incorporation d'un polymère acrylate (exemples comparatifs EC1 à EC8).

Ces mesures ont été effectuées suivant le protocole suivant :
on utilise un échantillon, réalisé comme décrit dans les exemples 1 à 8 et les exemples comparatifs 1 à 8, formé du support final, de la masse adhésive hydrophile et de la pellicule de papier siliconé qui sert de protecteur pelable, que l'on découpe de façon à obtenir un ruban adhésif.

La mesure s'effectue par différences de pesées entre la masse du ruban adhésif avant et après mise en contact, durant une durée déterminée, ici 24 heures avec un cylindre rempli d'un liquide de référence.

Dans les tests suivant le liquide de référence est une solution de Dextran D4876 (commercialisé par la société Sigma) à 60 g par litres dans une solution de chlorure de sodium à 0,15 molaire.

Les mesures sont réalisées suivant les étapes suivantes :
- on découpe un échantillon (ici par exemple de 16 cm²) du ruban adhésif à tester et on enlève le film protecteur.
- on place au centre de la partie à tester un cylindre en Teflon et on exerce une légère pression afin qu'il adhère correctement sur le ruban adhésif.
- l'ensemble ainsi obtenu est pesé ; soit P₀ le poids obtenu.
- on introduit alors 10 ml du liquide de référence précédemment préparé dans le cylindre.
- on laisse l'ensemble en contact à 23 °C pendant 24 heures.
- à l'issue des 24 heures on pèse, après élimination de la solution non absorbée, l'ensemble cylindre-ruban adhésif, soit P₁ le poids obtenu.
- on calcule le pouvoir absorbant qui correspond à l'absorption surfacique grâce à la formule : Absorption = 4(P₁-P₀)/πD²
où D représente le diamètre du cylindre soit ici 0,027 m.

D'où l'absorption exprimée en g/m² est définie ici par :
Absorption = (P₁-P₀)10⁴/5,73

Pour chaque test on réalise au moins 5 essais.

Le pouvoir absorbant obtenu est la moyenne de ces différents essais.

Les résultats obtenus ont été consignés dans le tableau I.

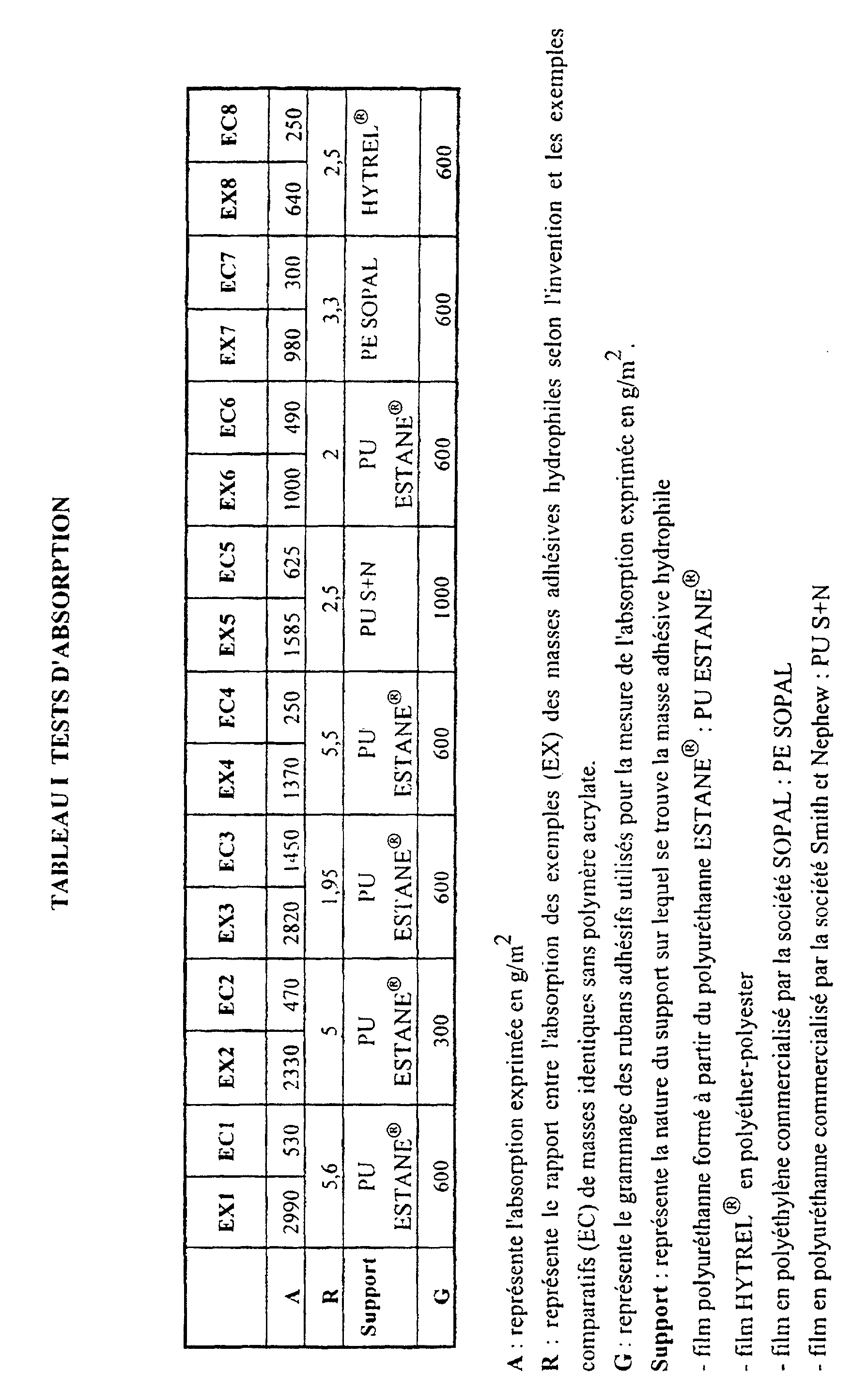

L'analyse des résultats regroupés au tableau I illustre parfaitement l'influence favorable de l'addition du polymère acrylate sur l'augmentation de la capacité d'absorption des masses adhésives hydrophiles selon l'invention. D'après le rapport R la capacité d'absorption des masses adhésives selon l'invention est toujours nettement supérieure à celle des exemples comparatifs, jusqu'à 5,6 fois supérieure dans le meilleur des cas.

On a ainsi une capacité d'absorption
- 5,6 fois supérieure pour l'exemple 1 par rapport à l'exemple comparatif EC1,
- 5 fois supérieure pour l'exemple 2 par rapport à l'exemple comparatif EC2,
- 2 fois supérieure pour l'exemple 3 par rapport à l'exemple comparatif EC3,
- 5,5 fois supérieure pour l'exemple 4 par rapport à l'exemple comparatif EC4,
- 2,5 fois supérieure pour l'exemple 5 par rapport à l'exemple comparatif EC5,
- 2 fois supérieure pour l'exemple 6 par rapport à l'exemple comparatif EC6,
- 3,3 fois supérieure pour l'exemple 7 par rapport à l'exemple comparatif EC7,
- 2,5 fois supérieur pour l'exemple 8 par rapport à l'exemple comparatif EC8.

On constate aussi que l'on retrouve cette augmentation pour tous les grammages testés 1000, 600 et 300 g/m².

Ainsi pour les exemples 1 et 2 le rapport R est quasi identique 5,6 et 5 respectivement.

On constate aussi que quelle que soit la nature des supports utilisés et donc leur perméabilité on trouve toujours une augmentation de la capacité d'absorption. Ceci est parfaitement illustré par les exemples 7 et 8, voire l'exemple 5.

De même quelle que soit la nature et le pourcentage de polymère acrylate utilisé (5 % d'ACRONAL® 3458 dans les exemples 1 à 5 ; 5 % d'ACRONAL® A150F dans l'exemple 6 ; et 3 et 8 % d'ACRONAL® 3429 respectivement dans les exemples 7 et 8), on constate toujours une augmentation significative de la capacité d'absorption puisqu'elle est au minimum doublée.

Enfin même si on fait varier la nature de la carboxyméthylcellulose de sodium (granulométrie différente dans l'exemple 3), ou la nature des polymères poly(SIS) dans la composition (KRATON® D1111CS pour les exemples 5 à 8 et VECTOR® 4114 pour les exemples 1 à 4), voire leurs proportions, on conserve toujours l'augmentation du pouvoir absorbant.

En conclusion toutes ces constatations et ces résultats démontrent de façon indéniable qu'au niveau de la composition de la masse adhésive hydrophile on possède une marge de manoeuvre relativement importante malgré l'introduction d'un composé supplémentaire, le polymère acrylate, pour réaliser un produit qui possède un pouvoir d'absorption nettement amélioré. Ceci est un avantage particulièrement important pour la réalisation de pansements protecteurs et pour le traitement de l'ampoule, des lésions dermo-épidermiques superficielles, des plaies et brûlures qui utilisent ces masses adhésives hydrophiles.

## Revendications

1. Masse adhésive hydrophile utilisable à des fins médicales **caractérisée en ce que** ladite masse adhésive hydrophile comprend :
**(a)** 10 à 35 parties en poids d'un copolymère séquencé poly(styrène-oléfinestyrène), en particulier poly(styrène-isoprène-styrène),
**(b)** 20 à 50 parties en poids d'une résine tackifiante,
**(c)** 2 à 15 parties en poids d'un polymère acrylate ayant une température de transition vitreuse inférieure à -20 °C,
**(d)** 2 à 25 parties en poids d'un plastifiant, notamment d'une huile plastifiante,
**(e)** 20 à 50 parties en poids d'un hydrocolloïde,
**(f)** 0.1 à 2 parties en poids d'au moins un agent antioxydant.

2. Masse adhésive hydrophile selon la revendication 1, **caractérisée en ce que** le polymère acrylate ayant une température de transition vitreuse inférieure à - 20 °C est un copolymère formé à partir d'au moins un monomère, choisi dans l'ensemble constitué par les esters alkylés de l'acide acrylique dans lesquels le groupe alkyle, linéaire ou ramifié, de l'ester comprend 1 à 18 atomes de carbone de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle d'isooctyle, de n-décyle et n-dodécyle, copolymérisé avec l'acide acrylique.

3. Masse adhésive hydrophile selon la revendication 2, **caractérisée en ce que** le copolymère acrylate précité est un copolymère formé à partir d'au moins un monomère, choisi dans l'ensemble constitué par l'acrylate de n-butyle, l'acrylate de 2-éthylhexyle et l'acrylate d'isooctyle, copolymérisé avec l'acide acrylique et de préférence un copolymère de n-butylacrylate et d'acide acrylique ayant une température de transition vitreuse de -39 °C ou de n-butylacrylate, 2-éthylhexyle acrylate et d'acide acrylique ayant une température de transition vitreuse de -31 °C.

4. Masse adhésive hydrophile selon la revendication 3, **caractérisée en ce que** le copolymère acrylate précité comprend de 1 à 20 %, de préférence 1 à 10 %, en poids d'acide acrylique exprimé par rapport au poids total de l'ensemble des monomères.

5. Masse adhésive hydrophile selon la revendication 1, **caractérisée en ce que** le polymère acrylate ayant une température de transition vitreuse inférieure à -20 °C est un copolymère formé à partir d'au moins ceux monomères, choisis dans l'ensemble constitué par les esters alkylés de l'acide acrylique dans lesquels le groupe alkyle, linéaire ou ramifié, de l'ester comprend 1 à 18 atomes de carbone de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle d'isooctyle, de n-décyle et n-dodécyle.

6. Masse adhésive hydrophile selon la revendication 1, **caractérisée en ce que** le polymère acrylate adhésif ayant une température de transition vitreuse inférieure à -20 °C est un homopolymére dont le monomère constitutif est choisi dans l'ensemble constitué par les esters alkylés de l'acide acrylique dans lesquels le groupe alkyle de l'ester est soit un groupe alkyle linéaire qui comprend 2 à 12 atomes de carbone, soit un groupe isobutyle, 2-éthylhexyle ou isooctyle et de préférence un homopolymère d'acrylate de n-butyle ayant une température de transition vitreuse de -41 °C.

7. Masse adhésive hydrophile selon l'une des revendications I à 6, **caractérisée en ce que** le copolymère séquencé précité est un poly(styrène-isoprène-styrène) qui présente une teneur en styrène comprise entre 14 et 52 % en poids par rapport au poids dudit copolymère, et de préférence une teneur comprise entre 14 et 30 % en poids.

8. Masse adhésive hydrophile selon l'une des revendications 1 à 7, **caractérisée en ce que** l'hydrocolloïde est un sel de métal alcalin de carboxyméthylcellulose, et de préférence la carboxyméthylcellulose de sodium.

9. Masse adhésive hydrophile selon l'une des revendications 1 à 8, **caractérisée en ce que** le plastifiant précité est une huile plastifiante minérale et de préférence une huile constituée de composés naphténiques, paraffiniques et aromatiques.

10. Masse adhésive hydrophile selon l'une des revendications 1 à 4, 7 à 9, **caractérisée en ce que**, pour un total de 100 parties en poids, elle comprend :
- 18 à 22, et de préférence 19,8 parties en poids d'un copolymère tribloc poly(styrène-isoprène-styrène),
- 25 à 35, et de préférence 30,2 parties en poids d'une résine tackifiante,
- 3 à 8, et de préférence 4,9 parties en poids d'un copolymère d'acrylate de n-butyle et d'acide acrylique qui présente une température de transition vitreuse de -39 °C,
- 10 à 20, et de préférence 14,3 parties en poids d'une huile plastifiante minérale,
- 25 à 35, et de préférence 30 parties en poids de carboxyméthylcellulose de sodium,
- 0,3 à 0,8, et de préférence 0,4 parties en pcids d'un agent antioxydant phénolique, et,
- 0,3 à 0,8, et de préférence 0,4 parties en poids d'ur agent antioxydant soufré, le dibutyldithiocarbamate de zinc.

11. Utilisation d'une masse adhésive hydrophile suivant l'une quelconque des revendications 1 à 10 pour la préparation d'un pansement destiné au traitement de l'ampoule, des lésions dermo-épidermiques superficielles de la peau, des plaies exsudatives et brûlures, formé d'un support sur lequel est enduit ladite masse adhésive hydrophile et éventuellement d'une pellicule de protection pelable.

## Claims

1. Hydrophilic adhesive mass which can be used for medical purposes, **characterised in that** said hydrophilic adhesive mass comprises:
**(a)** 10 to 35 parts by weight of a poly(styrene/olefin/styrene) block copolymer, particularly poly(styrene/isoprene/styrene),
**(b)** 20 to 50 parts by weight of a tackifying resin,
**(c)** 2 to 15 parts by weight of an acrylate polymer with a glass transition temperature below -20°C,
**(d)** 2 to 25 parts by weight of a plasticizer, especially a plasticizing oil,
**(e)** 20 to 50 parts by weight of a hydrocolloid, and
**(f)** 0.1 to 2 parts by weight of at least one antioxidant.

2. Hydrophilic adhesive mass according to claim 1, **characterised in that** the acrylate polymer with a glass transition temperature below -20°C is a copolymer formed from at least one monomer selected from the group consisting of acrylic acid alkyl esters in which the linear or branched alkyl group of the ester contains 1 to 18 carbon atoms, preferably 4 to 10 carbon atoms and particularly 4 to 8 carbon atoms, for example methyl, ethyl, n-propyl, n-butyl, isobutyl, n-hexyl, 2-ethylhexyl, n-octyl, isooctyl, n-decyl and n-dodecyl acrylates, copolymerised with acrylic acid.

3. Hydrophilic adhesive mass according to claim 2, **characterised in that** the above-mentioned acrylate copolymer is a copolymer formed from at least one monomer selected from the group consisting of n-butyl acrylate, 2-ethylhexyl acrylate and isooctyl acrylate, copolymerised with acrylic acid, and preferably an n-butyl acrylate/acrylic acid copolymer with a glass transition temperature of -39°C, or an n-butyl acrylate/2-ethylhexyl acrylate/acrylic acid copolymer with a glass transition temperature of -31°C.

4. Hydrophilic adhesive mass according to claim 3, **characterised in that** the above-mentioned acrylate copolymer comprises from 1 to 20% and preferably 1 to 10% by weight of acrylic acid, expressed relative to the total weight of all the monomers.

5. Hydrophilic adhesive mass according to claim 1, **characterised in that** the acrylate polymer with a glass transition temperature below -20°C is a copolymer formed from at least two monomers selected from the group consisting of acrylic acid alkyl esters in which the linear or branched alkyl group of the ester contains 1 to 18 carbon atoms, preferably 4 to 10 carbon atoms and particularly 4 to 8 carbon atoms, for example methyl, ethyl, n-propyl, n-butyl, isobutyl, n-hexyl, 2-ethylhexyl, n-octyl, isooctyl, n-decyl and n-dodecyl acrylates.

6. Hydrophilic adhesive mass according to claim 1, **characterised in that** the adhesive acrylate polymer with a glass transition temperature below -20°C is a homopolymer whose constituent monomer is selected from the group consisting of acrylic acid alkyl esters in which the alkyl group of the ester is either a linear alkyl group containing 2 to 12 carbon atoms or an isobutyl, 2-ethylhexyl or isooctyl group, and preferably an n-butyl acrylate homopolymer with a glass transition temperature of -41°C.

7. Hydrophilic adhesive mass according to one of claims 1 to 6, **characterised in that** the above-mentioned block copolymer is a poly(styrene/isoprene/styrene) with a styrene content of between 14 and 52% by weight, based on the weight of said copolymer, and preferably a content of between 14 and 30% by weight.

8. Hydrophilic adhesive mass according to one of claims 1 to 7, **characterised in that** the hydrocolloid is an alkali metal salt of carboxymethyl cellulose, preferably sodium carboxymethyl cellulose.

9. Hydrophilic adhesive mass according to one of claims 1 to 8, **characterised in that** the above-mentioned plasticizer is a mineral plasticizing oil, preferably an oil consisting of naphthenic, paraffinic and aromatic compounds.

10. Hydrophilic adhesive mass according to one of claims 1 to 4 and 7 to 9, **characterised in that** it comprises, for a total of 100 parts by weight:
- 18 to 22 and preferably 19.8 parts by weight of a poly(styrene/isoprene/styrene) three-block copolymer,
- 25 to 35 and preferably 30.2 parts by weight of a tackifying resin,
- 3 to 8 and preferably 4.9 parts by weight of an n-butyl acrylate/acrylic acid copolymer with a glass transition temperature of -39°C,
- 10 to 20 and preferably 14.3 parts by weight of a mineral plasticizing oil,
- 25 to 35 and preferably 30 parts by weight of sodium carboxymethyl cellulose,
- 0.3 to 0.8 and preferably 0.4 part by weight of a phenolic antioxidant, and
- 0.3 to 0.8 and preferably 0.4 part by weight of the sulphur-containing antioxidant zinc dibutyldithiocarbamate.

11. Use of a hydrophilic adhesive mass according to any one of claims 1 to 10 for the preparation of a dressing intended for the treatment of blisters, superficial dermo-epidermal lesions of the skin, exudative wounds and burns, said dressing being formed of a support onto which said hydrophilic adhesive mass is coated, and optionally of a peel-off protective film.

## Patentansprüche

1. Hydrophiler Klebstoff, verwendbar für medizinische Zwecke, **dadurch gekennzeichnet, dass** besagter hydrophiler Klebstoff umfasst:
(a) 10 bis 35 Gewichtsteile einer Copolymersequenz Poly- (Styrol-Olefin-Styrol), insbesondere Poly- (Styrol-Isopren-Styrol),
(b) 20 bis 50 Gewichtsteile eines klebrigen Harzes,
(c) 2 bis 15 Gewichtsteile eines Acrylatpolymers, mit einer Glasumwandlungstemperatur von unter -20°C,
(d) 2 bis 25 Gewichtsteile eines Weichmachers, insbesondere eines Weichmacheröls,
(e) 20 bis 50 Gewichtsteile eines Hydrokolloids
(f) 0,1 bis 2 Gewichtsteile zumindest eines Antioxidationsmittels.

2. Hydrophiler Klebstoff gemäß Anspruch 1, **dadurch gekennzeichnet, dass** das Acrylatpolymer mit einer Glasumwandlungstemperatur von unter 20°C ein Copolymer ist, das aus mindestens einem Monomer gebildet wird, ausgewählt aus der Gruppe bestehend aus den Alkylestern der Acrylsäure, in denen die Alkylgruppe des Esters, linear oder verzweigt, 1 bis 18 Kohlenstoffatome umfasst, vorzugsweise 4 bis 10 Kohlenstoffatome, insbesondere 4 bis 8 Kohlenstoffatome, wie zum Beispiel Methylacrylat, Ethylacrylat, n-Propylacrylat, n-Butylacrylat, Isobutylacrylat, n-Hexylacrylat, 2-Ethylhexylacrylat, iso-Octylacrylat, n-Octylacrylat, n-Dodecylacrylat, copolymerisiert mit Acrylsäure.

3. Hydrophiler Klebstoff, gemäß Anspruch 2, **dadurch gekennzeichnet, dass** das oben erwähnte Acrylatcopolymer ein Copolymer ist, das aus mindestens einem Monomer gebildet wird, das ausgewählt ist aus der Gruppe, bestehend aus n-Butylacrylat, 2-Ethylhexylacrylat, Isooctylacrylat, copolymerisiert mit Acrylsäure, und vorzugsweise ein Copolymer aus n-Butylacrylat und Acrylsäure, mit einer Glasumwandlungstemperatur von -39°C, oder aus n-Butylacrylat, 2-Ethylhexylacrylat und Acrylsäure, mit einer Glasumwandlungstemperatur von -31°C.

4. Hydrophiler Klebstoff gemäß Anspruch 3, **dadurch gekennzeichnet, dass** das oben erwähnte Acrylatcopolymer 1 bis 20 Gewichtsprozent, vorzugsweise 1 bis 10 Gewichtsprozent an Acrylsäure umfasst, bezogen auf das Gesamtgewicht der Gruppe der Monomere.

5. Hydrophiler Klebstoff gemäß Anspruch 1, **dadurch gekennzeichnet, dass** das Acrylatpolymer mit einer Glasumwandlungstemperatur von unter -20°C ein Copolymer ist, das aus mindestens zwei Monomeren gebildet ist, ausgewählt aus der Gruppe, bestehend aus den Alkylestern der Acrylsäure, in denen die Alkylgruppe des Esters, linear oder verzweigt, 1 bis 18 Kohlenstoffatome, vorzugsweise 4 bis 10 Kohlenstoffatome, insbesondere 4 bis 8 Kohlenstoffatome, umfasst, wie zum Beispiel das Methylacrylat, Ethylacrylat, n-Propylacrylat, n-Butylacrylat, Isobutylacrylat, n-Hexylacrylat, 2-Ethylhexylacrylat, Isooctylacrylat, n-Octylacrylat, n-Decylacrylat und n-Dodecylacrylat.

6. Hydrophiler Klebstoff gemäß Anspruch 1, **dadurch gekennzeichnet, dass** das klebrige Acrylatpolymer mit einer Glasumwandlungstemperatur von unter -20°C ein Homopolymer ist, dessen monomerer Bestandteil ausgewählt ist aus der Gruppe der Alkylester der Acrylsäure, bei denen die Alkylgruppe entweder eine lineare Alkylgruppe, die 2 bis 12 Kohlenstoffatome umfasst, oder eine Isobutyl-, 2-Ethylhexyl- oder Isooctylgruppe, vorzugsweise ein n-Butylacrylat-Homopolymer mit einer Glasumwandlungstemperatur von -41°C, ist.

7. Hydrophiler Klebstoff gemäß einem der Ansprüche 1 bis 6, **dadurch gekennzeichnet, dass** die oben erwähnte Copolymersequenz ein Poly-(Styrol-Isopren-Styrol) ist, das einen Styrolgehalt aufweist, der zwischen 14 und 52 Gewichtsprozent liegt, und vorzugsweise einen Gehalt, der zwischen 14 und 30 % des Gewichts, im Verhältnis zum Gewicht des genannten Copolymers, liegt.

8. Hydrophiler Klebstoff gemäß einem der Ansprüche 1 bis 7, **dadurch gekennzeichnet, dass** das Hydrokolloid ein Salz des Alkalimetalls der Carboxymethylcellulose, vorzugsweise der Natriumcarboxymethylcellulose, ist.

9. Hydrophiler Klebstoff gemäß einem der Ansprüche 1 bis 8, **dadurch gekennzeichnet, dass** der oben erwähnte Weichmacher ein Mineralöl-Weichmacher ist, vorzugsweise ein Öl, gebildet aus naphthenischen, paraffinischen und aromatischen Verbindungen.

10. Hydrophiler Klebstoff gemäß einem der Ansprüche 1 bis 4 und 7 bis 9, **dadurch gekennzeichnet, dass** er, bezogen auf insgesamt 100 Gewichtsteile, umfasst:
- 18 bis 22 und vorzugsweise 19,8 Gewichtsteile eines Tribloc-Copolymers Poly-(Styrol-Isopren-Styrol),
- 25 bis 35 und vorzugsweise 30,2 Gewichtsteile eines klebrigen Harzes,
- 3 bis 8 und vorzugsweise 4,9 Gewichtsteile eines n-Butylacrylat-Copolymers der Acrylsäure, mit einer Glasumwandlungstemperatur von -39°C,
- 10 bis 20 und vorzugsweise 14,3 Gewichtsteile eines Mineralöl-Weichmachers,
- 25 bis 35 und vorzugsweise 30 Gewichtsteile Natriumcarboxymethylcellulose,
- 0,3 bis 0,8 und vorzugsweise 0,4 Gewichtsteile eines phenolischen Antioxidationsmittels, und,
- 0,3 bis 0,8 und vorzugsweise 0,4 Gewichtsteile eines schwefelhaltigen Antioxidationsmittels, dem Zinkdibutyldithiocarbamat.

11. Verwendung eines hydrophilen Klebstoffs gemäß einem der Ansprüche 1 bis 10 zur Herstellung eines Verbands zur Behandlung von Druckblasen, oberflächlicher dermalepidermaler Läsionen der Haut, exsudativer Wunden und Verbrennungen, gebildet aus einem Träger, auf dem der besagte hydrophile Klebstoff aufgetragen ist, und gegebenenfalls einem abziehbaren Schutzfilm.
